# EUROPÄISCHE PATENTSCHRIFT

(11) **EP 2 787 957 B1**
(45) Veröffentlichungstag und Bekanntmachung des Hinweises auf die Patenterteilung: **15.05.2019**
(21) Anmeldenummer: 12781357.4
(22) Anmeldetag: 09.11.2012
(51) Int. Cl.: A61K 8/39, A61Q 5/06, A61K 8/86, A61K 8/04

(54) **TREIBMITTELHALTIGES KOSMETISCHES MITTEL**
PROPELLENT-CONTAINING COSMETIC COMPOSITION
PRODUIT COSMETIQUE CONTENANT UN AGENT PROPULSEUR

(30) Priorität: 09.12.2011 DE 102011088148
(43) Veröffentlichungstag der Anmeldung: 15.10.2014
(73) Patentinhaber: Henkel AG & Co. KGaA, 40589 Düsseldorf (DE)
(72) Erfinder: MÜLLER, Burkhard, 21075 Hamburg (DE); THAMMASIRI, Anja, 25336 Klein Nordende (DE)
(86) Internationale Anmeldenummer: PCT/EP2012/072244
(87) Internationale Veröffentlichungsnummer: WO 2013/083350

(56) Entgegenhaltungen:
- EP-A1- 1 792 600
- EP-A2- 2 098 217
- WO-A1-2010/126090
- WO-A1-2013/048779
- CA-A1- 2 571 285
- GB-A- 1 121 563

## Beschreibung

Die vorliegende Erfindung betrifft kosmetische Mittel mit Treibmitteln.

Gesundes Haar und eine ansprechend aussehende Frisur wird heute allgemein als unverzichtbarer Teil eines gepflegten Äußeren angesehen. Im Allgemeinen empfindet der Mensch stumpfes Haar oder dünnes Haar als ästhetischen Makel. Wird das Haar Umwelteinflüssen wie z.B. UV-Strahlung, mechanischer Belastung oder freien Radikalen ausgesetzt, kann die Haarstruktur negativ beeinträchtigt werden. Das wirkt sich auf das äußere Erscheinungsbild aus. Beim so genannten Haarspliss z.B. sind die Haarspitzen in ihrer Haarstruktur sichtbar degeneriert und lassen das Haar krank und ungesund aussehen. Mit natürlichem Glanz und Haarfülle assoziiert der Mensch ein Schönheitsideal, das er für sich in Anspruch zu nehmen sucht. Ferner möchte er das Haar in eine ihm gefällige Form (Frisur) bringen und diese Form fixieren. Als Hilfsmittel dienen dabei kosmetische Mittel, welche die Haare schützen oder die Haare in ihren Eigenschaften derart verändern, dass das sich das Aussehen des Haars dem gewünschten Schönheitsideal annähert.

Entsprechende Mittel zur temporären Formgebung enthalten als formgebende Komponente üblicherweise festigende Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die Anwendung als mittels Treibgas aufgeschäumtes Kosmetikum findet große Akzeptanz, da sich Schäume einfach und gleichmäßig auf dem Haar verteilen lassen. Allerdings müssen die Schäume ein besonderes Anforderungsprofil erfüllen, damit die Applikation optimal durchgeführt werden kann und sich die Wirkung der Polymere auf dem Haar entsprechend entfalten kann. Die Schäume müssen sich rasch bilden, eine kurze Zeit stabil bleiben und auf dem Haar brechen, damit keine dauerhafte Schaumhaube entsteht. Der Schaum darf das Haar nicht belasten, ansonsten hängt sich die aufgeprägte Frisur aus und geht dadurch verloren.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten festigenden Polymers bestimmt, wobei jedoch auch ein Einfluss der weiteren Bestandteile des Stylingmittels und der Applikationsart gegeben sein kann.

Neben einem hohen Haltegrad müssen Stylingmittel eine ganze Reihe weiterer Anforderungen erfüllen. Zu nennen sind insbesondere Feuchtebeständigkeit, niedrige Klebrigkeit und ein ausgewogener Konditioniereffekt. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hatte Schuppen.

Aufgabe der vorliegenden Erfindung war es daher, ein kosmetisches Mittel mit Treibgas zur Verfügung zu stellen, das sich durch einen hohen Haltegrad auszeichnet. Weiterhin soll das Mittel gleichzeitig einen flexiblen Halt aufweisen sowie eine hohe Pflegeleistung zeigen. Die Bildung von Filmplaken - insbesondere die Bildung entsprechender Rückstände bei der Anwendung, dem Ausbürsten und der Trocknung der keratinhaltigen Faser - soll vermieden werden. Werden die Mittel als Schaum ausgestaltet soll der Schaum sich schnell bilden, erst bei Auftragung am Haar beginnen signifikant zu brechen. Ferner soll ein Schaum bereitgestellt werden, der das Haar nicht belastet.

Es wurde nunmehr gefunden, dass dies durch eine Kombination zweier spezieller nichtionischer Tenside erreicht wird.

Ein erster Gegenstand der Erfindung ist daher ein kosmetisches Mittel zur kosmetischen Behandlung keratinischer Fasern, insbesondere menschlicher Haare, enthaltend in einem kosmetischen Träger
i) mindestens ein festigendes Polymer, ausgewählt aus der Gruppe, die gebildet wird aus nichtionischen, festigenden Polymeren und kationischen, festigenden Polymeren, und
ii) 0,05 bis 0,4 Gew.-% - bezogen auf das Gesamtgewicht des Mittels - an Verbindungen der Formel (I), umfassend höchstens 20 Ethylenoxideinheiten,

   R¹-X¹-(O-CH₂CH₂)n-OH (I)

   worin
   R¹ steht für einen linearen oder verzweigten, gesättigten oder ungesättigten Kohlenwasserstoffrest mit 8 bis 20 Kohlenstoffatomen,
   X¹ steht für eine direkte Bindung oder eine Carbonylgruppe,
   n steht für eine ganze Zahl von 1 bis 20,
   und
iii) mindestens eine lineare oder verzweigte Verbindung der Formel (II) mit mindestens 30 Ethylenoxideinheiten 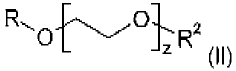 worin
   R² für eine lineare oder verzweigte (C₇ bis C₂₁)-Alkylcarbonylgruppe, eine lineare oder verzweigte (C₇ bis C₂₁)-Alkenylcarbonylgruppe oder für einen linearen oder verzweigten, gesättigten oder ungesättigten (C₈ bis C₂₂)-Kohlenwasserstoffrest steht,
   R steht für ein Wasserstoffatom oder eine Gruppe der Formel (III) 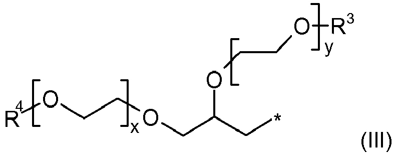
      in der x und y unabhängig voneinander für eine ganze Zahl von 0 bis 80 stehen,
      R³ und R⁴ unabhängig voneinander für ein Wasserstoffatom oder eine lineare oder verzweigte, gesättigte oder ungesättigte (C₇ bis C₂₁)-Alkylcarbonylgruppe stehen,
   z steht für eine ganze Zahl von 0 bis 80
   mit den Maßgaben, dass
      - wenn R eine Gruppe der Formel (III) ist, mindestens eine Gruppe aus R², R³, R⁴ für eine lineare oder verzweigte (C₇ bis C₂₁)-Alkylcarbonylgruppe oder eine lineare oder verzweigte (C₇ bis C₂₁)-Alkenylcarbonylgruppe steht und die Summe x + y + z mindestens 30 beträgt,
      - wenn R für ein Wasserstoffatom steht z eine ganze Zahl von 30 bis 80 bedeutet
   und
iv) mindestens ein Treibmittel.

Unter keratinischen Fasern sind dabei erfindungsgemäß Pelze, Wolle, Federn und insbesondere menschliche Haare zu verstehen.

Gemäß obigen Formeln und allen folgenden Formeln steht eine chemische Bindung, die mit dem Symbol * gekennzeichnet ist, für eine freie Valenz des entsprechenden Strukturfragments.

Unter Polymeren werden erfindungsgemäß Verbindungen verstanden, die von den Verbindungen der Formeln (I) und (II) verschieden sind und die ein Molekulargewicht von wenigstens 10000 g/mol besitzen. Die Polymere sind aus einer Vielzahl von Molekülen aufgebaut, in denen eine Art oder mehrere Arten von Atomen oder Atomgruppierungen (sogenannte konstitutive Einheiten, Grundbausteine oder Wiederholungseinheiten) wiederholt aneinander gereiht sind. Die Polymere werden durch Polyreaktion erhalten, wobei letztere künstlich (d.h. synthetisch) oder natürlich erfolgen kann.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden.

Unter filmbildenden Polymeren werden weiterhin solche Polymere verstanden, die bei Anwendung in 0,01 bis 20 Gew.-%-iger wässriger, alkoholischer oder wassrigalkoholischer Lösung in der Lage sind, auf dem Haar einen transparenten Film abzuscheiden.

Die festigenden Polymere sind bevorzugt in einer Menge von 0,1 Gew.-% bis 15,0 Gew.-%, insbesondere von 1,0 Gew.-% bis 12,0 Gew.-%, ganz besonders bevorzugt von 2,0 bis 10,0 Gew.-%, jeweils bezogen auf das Gewicht des kosmetischen Mittels, enthalten

Im Rahmen einer besonders bevorzugten Ausführungsform enthalten die erfindungsgemäßen Mittel als festigendes Polymer mindestens ein festigendes, nichtionisches Polymer.

Die festigenden, nichtionischen Polymere sind in dem erfindungsgemäßen Mittel bevorzugt in einer Menge von 0,1 Gew.-% bis 10,0 Gew.-%, besonders bevorzugt von 1,0 Gew.-% bis 8,0 Gew.-%, ganz besonders bevorzugt von 2,0 Gew.-% bis 6,0 Gew.-%, jeweils bezogen auf das Gewicht der erfindungsgemäßen Mittel, enthalten.

Die festigenden, nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens,
- nichtionischen Copolymeren des Maleinsäureanhydrids.

Geeignete Polyvinylpyrrolidone sind beispielsweise Handelsprodukte wie Luviskol® K 90 oder Luviskol® K 85 der Firma BASF SE.

Geeignetes Polyvinylacetat wird beispielsweise unter dem Handelsnamen Vinac® als Emulsion von der Firma Air Products vertrieben.

Mittel, die als festigendes, nichtionisches Polymer mindestens ein Polymer ausgewählt aus der Gruppe, die gebildet wird aus
- Copolymer aus Maleinsäureanhydrid und Methylvinylether,
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   oder Gemische aus diesen Polymeren enthalten, sind erfindungsgemäß ganz besonders bevorzugt. Dabei sind wiederum solche erfindungsgemäßen Mittel bevorzugt, die als festigendes, nichtionisches Polymer mindestens ein Polymer ausgewählt aus der Gruppe, die gebildet wird aus

- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   oder Gemische aus diesen Polymeren enthalten.

Wenn Copolymere aus N-Vinylpyrrolidon und Vinylestern von Carbonsäuren mit 2 bis 18 Kohlenstoffatomen, insbesondere aus N-Vinylpyrrolidon und Vinylacetat eingesetzt werden, ist es wiederum bevorzugt, wenn das Molverhältnis der aus dem Monomer N-Vinylpyrrolidon enthaltenen Struktureinheiten zu den aus dem Monomer Vinylestern von Carbonsäuren mit 2 bis 18 Kohlenstoffatomen (insbesondere Vinylacetat) enthaltenen Struktureinheiten des Polymers im Bereich von 20 zu 80 bis 80 zu 20, insbesondere von 30 zu 70 bis 70 zu 30, liegt.

Geeignete Copolymerisate aus Vinylpyrrolidon und Vinylacetat sind beispielsweise unter dem Warenzeichen Luviskol® VA 37, Luviskol® VA 55, Luviskol® VA 64 und Luviskol® VA 73 von der Firme BASF SE erhältlich.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein erfindungsgemäß bevorzugt geeignetes festigendes, kationisches Polymer ist mindestens ein festigendes, kationisches Polymer, das mindestens ein Strukturelement der Formel (M9) und zusätzlich mindestens ein Strukturelement der Formel (M10) enthält 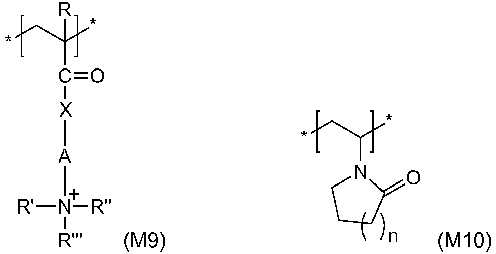 worin
- R: für ein Wasserstoffatom oder eine Methylgruppe steht,
- R', R" und R": unabhängig voneinander stehen für eine (C₁ bis C₃₀)-Alkylgruppe,
- X: steht für ein Sauerstoffatom oder eine Gruppe NH,
- A: steht für eine Ethan-1,2,-diylgruppe oder eine Propan-1,3-diylgruppe,
- n: 1 oder 3 bedeutet.

Weiterhin eignet sich als besonders bevorzugt geeignetes festigendes, kationisches Polymer mindestens ein amphiphiles, kationisches Polymer, umfassend mindestens eine Struktureinheit der Formel (M6) und mindestens eine Struktureinheit der Formel (VI) und gegebenenfalls mindestens eine Struktureinheit der Formel (V) 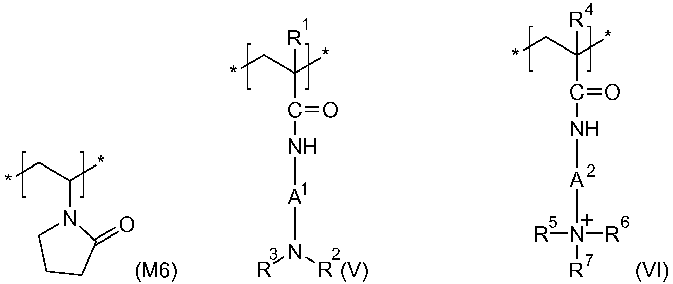 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Es ist erfindungsgemäß bevorzugt, wenn ein erfindungsgemäßes Mittel als obiges geeignetes festigendes, kationisches Polymer mindestens ein amphiphiles, kationisches Polymer, umfassend mindestens eine Struktureinheit der Formel (M6), mindestens eine Struktureinheit der Formel (M7), mindestens eine Struktureinheit der Formel (III) und mindestens eine Struktureinheit der Formel (IV), 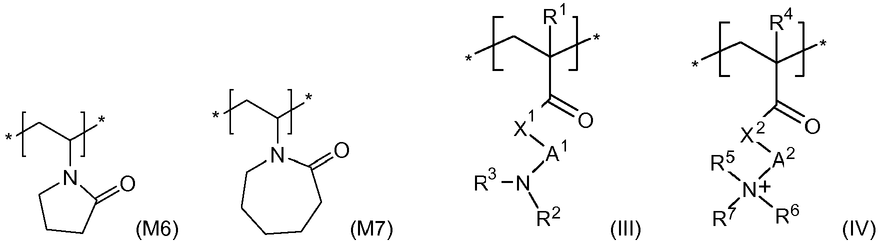 worin
R¹ und R⁴ stehen unabhängig voneinander für ein Wasserstoffatom oder eine Methylgruppe,
X¹ und X² stehen unabhängig voneinander für ein Sauerstoffatom oder eine Gruppe NH,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe
enthält.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Solche Verbindungen sind beispielsweise als
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),
- Copolymere aus N-Vinylpyrrolidon, N-(3-Dimethylaminopropyl)methacrylamid und 3-(Methacryloylamino)propyl-lauryl-dimethylammoniumchlorid (INCI-Bezeichnung: Polyquaternium-55), welches beispielsweise unter dem Handelsnamen Styleze W 10 oder W 20 (W 10: 10 Gew.-% Aktivsubstanz bzw. W 20: 20 Gew.-% Aktivsubstanz, jeweils in Ethanol-Wasser-Gemisch) von der Firma ISP vertrieben wird.
- Copolymere aus N-Vinylpyrrolidon, N-Vinylcaprolactam, N-(3-Dimethylaminopropyl)methacrylamid und 3-(Methacryloylamino)propyl-lauryldimethylammoniumchlorid (INCI-Bezeichnung: Polyquaternium-69), welches beispielsweise unter dem Handelsnamen AquaStyle® 300 (28-32 Gew.-% Aktivsubstanz in Ethanol-WasserGemisch) von der Firma ISP vertrieben wird.

Weiterhin werden die kationischen filmbildenden und/oder kationischen festigenden Polymere erfindungsgemäß besonders bevorzugt aus kationischen, quaternisierten Cellulose-Derivaten ausgewählt.

Ferner eignen sich als filmbildendes und/oder festigendes Polymere bevorzugt kationische, quaternisierte Cellulosederivate.

Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100, Celquat® L 200 von der Firma National Starch vertrieben werden.

Als im Sinne der Erfindung besonders bevorzugt verwendbare kationische Polymere dienen weiterhin solche kationischen festigenden Copolymere, die mindestens ein Strukturelement der Formel (M11) 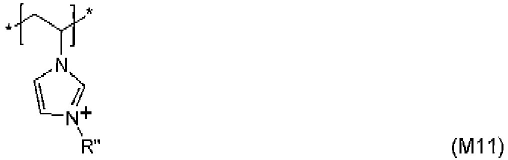
worin R" für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als kationisches festigendes Polymer, mindestens ein Copolymer (c1) enthalten ist, das neben mindestens einem Strukturelement der Formel (M11) zusätzlich ein Strukturelement der Formel (M6) umfasst 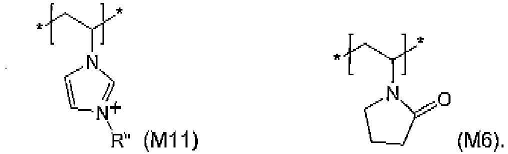 worin R" für eine (C₁ bis C₄)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat® FC 550, Luviquat® FC 905 und Luviquat® HM 552 erhältlich.

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat® UltraCare erhältlich.

Zusätzlich zu dem bzw. den Copolymer(en) (c1) oder an dessen bzw. deren Stelle können die erfindungsgemäßen pulverförmigen Zusammensetzungen auch Copolymere (c2) enthalten, die ausgehend vom Copolymer (c1) als zusätzliche Struktureinheiten Struktureinheiten der Formel (M7) aufweisen

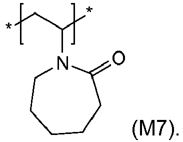

Weitere besonders bevorzugte erfindungsgemäße pulverförmige Zusammensetzungen sind somit dadurch gekennzeichnet, daß sie als kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

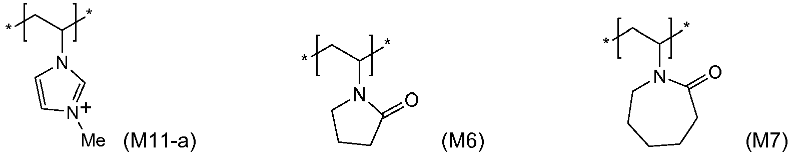

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat® Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Zusätzlich zu dem bzw. den Copolymer(en) (c1) und/oder (c2) oder an dessen bzw. deren Stelle können die erfindungsgemäßen pulverförmigen Zusammensetzungen als festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als kationisches filmbildendes und/oder kationisches festigendes Polymer mindestens ein Copolymer (c1) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Forme! (M12) enthält

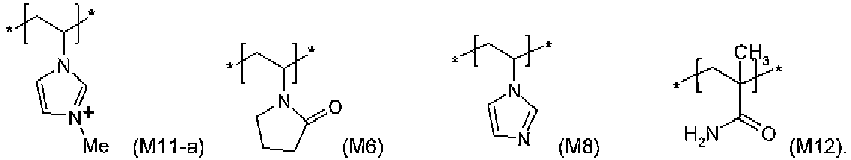

Auch hierbei ist besonders bevorzugt, wenn die Copolymere (c1) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11-a), (M6), (M8) und (M12) aufgebaut.

Zur Kompensation der positiven Polymerladung der Komponente (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat® Supreme erhältlich.

Weitere in den erfindungsgemäßen Mitteln bevorzugt einsetzbare kationische, festigende Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Als temporär kationische Polymere stellen Chitosane festigende, kationische Polymere dar. Chemisch betrachtet, handelt es sich bei Chitosanen um partiell deacetylierte Chitine unterschiedlichen Molekulargewichtes.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, die ein mittleres Molekulargewicht (Gewichtsmittel) von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Neben den Chitosanen als typische Biopolymere kommen im Sinne der Erfindung auch kationisch derivatisierte Chitosane (wie z. B. Quaternierungsprodukte) oder alkoxylierte Chitosane als Derivate des Chitosans in Frage.

Erfindungsgemäß bevorzugte Mittel sind dadurch gekennzeichnet, daß sie als festigendes, kationisches Polymer mindestens ein Neutralisationsprodukt von Chitosan mit mindestens einer organischen Carbonsäure, wie insbesondere Ameisensäure, Essigsäure, Zitronensäure, Milchsäure, Pyrrolidoncarbonsäure, Weinsäure, Glycolsäure, Nicotinsäure, Hydroxyisobuttersäure, Hydroxyisovaleriansäure oder Gemischen dieser Säuren, enthalten. Hierbei ist es erfindungsgemäß bevorzugt, die organische Carbonsäure unter Milchsäure, Ameisensäure, Pyrrolidoncarbonsäure, Nicotinsäure, Hydroxyisobuttersäure, Hydroxyisovaleriansäure oder Gemischen dieser Säuren auszuwählen. Dieses Neutralisationsprodukt kann beispielsweise in einem wässrigen Medium durch Zugabe von Chitosan und der entsprechenden organischen Carbonsäure hergestellt werden.

Geeignete Chitosane sind beispielsweise unter den Handelsbezeichnungen Hydagen® CMF (1 Gew.-% Aktivsubstanz in wässriger Lösung mit 0.4 Gew.-% Glykolsäure, Molekulargewicht 500000 bis 5000000 g/mol Cognis), Hydagen® HCMF (Chitosan (zu 80 % deacetyliert), Molekulargewicht 50000 bis 1000000 g/mol, Cognis), Kytamer® PC (80 Gew.-% Aktivsubstanz an Chitosan pyrolidoncarboxylat (INCI-Bezeichnung: Chitosan PCA), Amerchol) und Chitolam® NB/101 im Handel frei verfügbar.

Die Chitosane bzw. deren Derivate sind in den erfindungsgemäßen Mitteln bevorzugt in einer Menge von 0,01 Gew.-% bis 5,0 Gew.-%, besonders bevorzugt von 0,05 Gew.-% bis 2,0 Gew.-%, ganz besonders bevorzugt von 0,1 Gew.-% bis 1,0 Gew.-%, jeweils bezogen auf das Gewicht des erfindungsgemäßen Mittels, enthalten.

Als im Sinne der Erfindung bevorzugt geeignete temporär kationische Polymere gelten gleichfalls solche, die nicht permanent kationisch sind und mindestens eine Struktureinheit der Formeln (M1-1) bis (M1-8) aufweisen

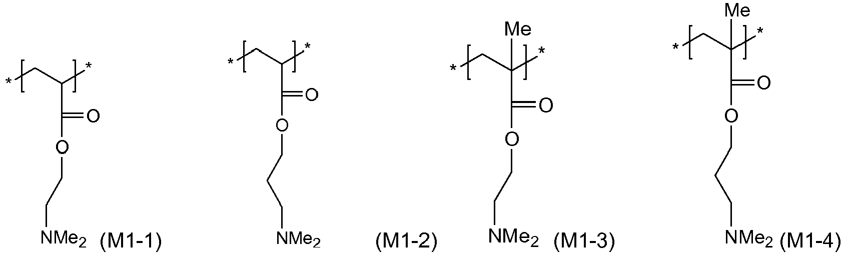

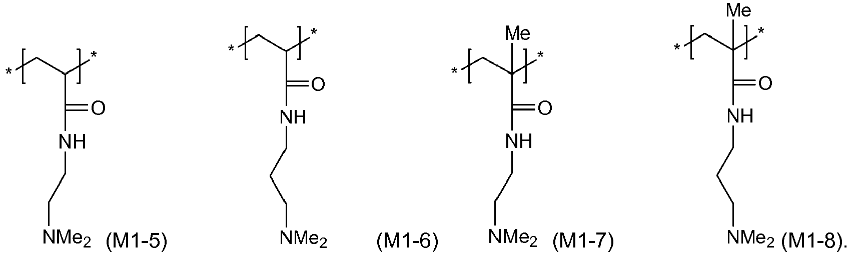

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten, 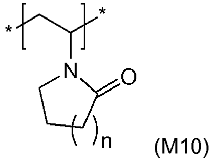 worin
- n: 1 oder 3 bedeutet.

Dabei gilt wiederum die Gruppe der Polymere
- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix® VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex® SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),
als bevorzugte Liste zur Auswahl mindestens eines oder mehrerer Polymere daraus.

Bevorzugte kosmetische Mittel enthalten solche Verbindungen der Formel (I), bei denen X¹ für eine direkte Bindung steht.

Ferner hat es sich als bevorzugt erwiesen, wenn die Verbindungen der Formel ausgewählt werden aus solchen, bei denen n gemäß Formel (I) für eine ganze Zahl von 1 bis 10 steht.

Der erfindungsgemäße Effekt lässt sich weiterhin optimieren, in dem die in dem erfindungsgemäßen Mittel enthaltenen Verbindungen der Formel (I) ein Molekulargewicht von maximal 1200 g/mol, insbesondere maximal 760 g/mol, besitzen.

Bevorzugte Verbindungen der Formel (I) werden ausgewählt unter mindestens einer Verbindung der Gruppe, die gebildet wird aus Oleylalkohol mit 2 Moläquivalenten Ethylenoxid (Oleth-2), Oleylalkohol mit 5 Moläquivalenten Ethylenoxid (Oleth-5), Oleylalkohol mit 10 Moläquivalenten Ethylenoxid (Oleth-10), Stearylalkohol mit 10 Aquivalenten Ethylenoxid (Steareth-10), Cetearylalkohol mit 6 Moläquivalenten Ethylenoxid (Ceteareth-6), Cetearylalkohol mit 12 Moläquivalenten Ethylenoxid (Ceteareth-12), Laurylalkohol mit 2 Äquivalenten Ethylenoxid (Laureth-2), Laurylalkohol mit 3 Äquivalenten Ethylenoxid (Laureth-3), Laurylalkohol mit 4 Äquivalenten Ethylenoxid (Laureth-4), Laurylalkohol mit 10 Äquivalenten Ethylenoxid (Laureth-10), Caprylalkohol mit 4 Aquivalenten Ethylenoxid (Capryleth-4).

Weiterhin zeigt sich, dass bevorzugterweise die in dem erfindungsgemäßen Mittel enthaltenen Verbindungen der Formel (II) ein Molekulargewicht von mindestens 1450 g/mol, insbesondere mindestens 1550 g/mol, besitzen.

Es ist erfindungsgemäß bevorzugt, wenn die Verbindungen gemäß Formel (II) ausgewählt werden aus mindestens einer Verbindung der Formel (II-a) 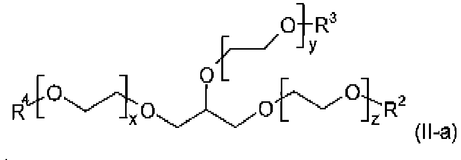 worin
R², R³ und R⁴ stehen unabhängig voneinander für ein Wasserstoffatom, eine lineare oder verzweigte (C₇ bis C₂₁)-Alkylcarbonylgruppe, oder eine lineare oder verzweigte (C₇ bis C₂₁)-Alkenylcarbonylgruppe,
x, y und z stehen unabhängig voneinander für eine ganze Zahl von 0 bis 80
mit den Maßgaben, dass
   - mindestens eine Gruppe aus R², R³, R⁴ von einem Wasserstoffatom verschieden ist und
   - die Summe x + y + z mindestens 30 beträgt
Bevorzugte Vertreter dieser Verbindungen der Formel (II-a) sind hydrogenierte Triglyzeride, welche 30 bis 80 Moläquivalent Ethylenoxideinheiten enthalten. Solche bevorzugten hydrogenierte und ethoxylierte Triglyzeride werden besonders bevorzugt ausgewählt aus mindestens einer Verbindung aus der Gruppe, die gebildet wird aus PEG-40 Hydrogenated Castor Oil, PEG-50 Hydrogenated Castor Oil, PEG-54 Hydrogenated Castor Oil, PEG-60 Hydrogenated Castor Oil, PEG-40 Castor Oil, PEG-50 Castor Oil, PEG-54 Castor Oil, PEG-60 Castor Oil.

Bezogen auf das Gesamtgewicht der erfindungsgemäßen Mittel sind die Verbindungen der Formel (II) bevorzugt in einer Menge von 0,05 bis 0,4 Gew.-% enthalten.

Bevorzugte kosmetische Mittel sind dadurch gekennzeichnet, dass die Menge der Verbindungen der Formel (I) und der Verbindungen der Formel (II) in einem Gewichtsverhältnisbereich von 4 zu 1 bis 1 zu 4, insbesondere von 3 zu 1 bis 1 zu 3, liegt.

Ein bevorzugtes erfindungsgemäßes kosmetisches Mittel zur kosmetischen Behandlung keratinischer Fasern, insbesondere menschlicher Haare, enthält, bezogen auf das Gewicht des gesamten Mittels, in einem kosmetischen Träger
i) 0,1 Gew.-% bis 15,0 Gew.-%, insbesondere 1,0 Gew.-% bis 12,0 Gew.-%, ganz besonders bevorzugt 2,0 bis 10,0 Gew.-%, mindestens eines festigenden Polymers, ausgewählt aus der Gruppe, die gebildet wird aus nichtionischen, festigenden Polymeren und kationischen, festigenden Polymeren, und
ii) 0,05 bis 0,4 Gew.-% mindestens einer Verbindung der Formel (I), umfassend höchstens 20 Ethylenoxideinheiten,

   R¹-X¹-(O-CH₂CH₂)ₙ)-OH (I)

   worin
   R¹ steht für einen linearen oder verzweigten, gesättigten oder ungesättigten Kohlenwasserstoffrest mit 8 bis 20 Kohlenstoffatomen,
   X¹ steht für eine direkte Bindung oder eine Carbonylgruppe,
   n steht für eine ganze Zahl von 1 bis 20,
   und
iii) 0,05 bis 0,4 Gew.-% mindestens einer linearen oder verzweigten Verbindung der Formel (II) mit mindestens 30 Ethylenoxideinheiten 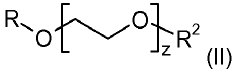 worin
   R² für eine lineare oder verzweigte (C₇ bis C₂₁)-Alkylcarbonylgruppe, eine lineare oder verzweigte (C₇ bis C₂₁)-Alkenylcarbonylgruppe oder für einen linearen oder verzweigten, gesättigten oder ungesättigten (C₈ bis C₂₂)-Kohlenwasserstoffrest steht,
   R steht für ein Wasserstoffatom oder eine Gruppe der Formel (III) 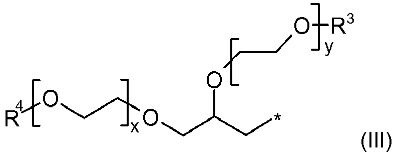
      in der x und y unabhängig voneinander für eine ganze Zahl von 0 bis 80 stehen,
      R³ und R⁴ unabhängig voneinander für ein Wasserstoffatom oder eine lineare oder verzweigte, gesättigte oder ungesättigte (C₇ bis C₂₁)-Alkylcarbonylgruppe stehen,
   z steht für eine ganze Zahl von 0 bis 80
   mit den Maßgaben, dass
      - wenn R eine Gruppe der Formel (III) ist, mindestens eine Gruppe aus R², R³, R⁴ für eine lineare oder verzweigte (C₇ bis C₂₁)-Alkylcarbonylgruppe oder eine lineare oder verzweigte (C₇ bis C₂₁)-Alkenylcarbonylgruppe steht und die Summe x + y + z mindestens 30 beträgt,
      - wenn R für ein Wasserstoffatom steht z eine ganze Zahl von 30 bis 80 bedeutet
   und
iv) 1,0 bis 35,0 Gew.-% mindestens eines Treibmittels (insbesondere Propan und/oder Butan).

Die erfindungsgemäßen Mittel enthalten ihre Wirkstoffe in einem kosmetischen Träger, bevorzugt in einem wasserhaltigen kosmetischen Träger, alkoholischen kosmetischen Träger oder einem wässrig-alkoholischen kosmetischen Träger. Es ist erfindungsgemäß bevorzugt, wenn es sich um einen wasserhaltigen kosmetischen Träger oder einen wässrig-alkoholischen kosmetischen Träger handelt. Es ist erfindungsgemäß bevorzugt, wenn der kosmetische Träger des erfindungsgemäßen Mittels Wasser enthält. Besonders bevorzugt enthält das erfindungsgemäße Mittel mindestens 50 Gew.-% Wasser - bezogen auf das Gewicht des gesamten Mittels.

Unter wässrig-alkoholischen Trägern sind im Sinne der vorliegenden Erfindung wässrige Zusammensetzungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol, 1,2-Propylenglykol oder 1,3-Propylenglykol enthalten. Bevorzugt sind dabei alle wasserlöslichen organischen Lösemittel.

Weiterhin kann das erfindungsgemäße Mittel zur Steigerung der Haarpflegewirkung zusätzlich mindestens ein kationisches Tensid enthalten. Als kationische Tenside werden im Sinne der Erfindung keine Polymere verstanden. Beispiele für die in den erfindungsgemäßen Mitteln einsetzbaren kationischen Tenside sind insbesondere quartären Ammoniumverbindungen mit ein oder zwei (C₈ bis C₂₀)-Alkylresten. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, sogenannte "Esterquats", wie beispielsweise die unter den Warenzeichen Dehyquart® und Stepantex® vertriebenen Methyl-hydroxyalkyldialkoyloxyalkyl-ammoniummethosulfate, als besagte quartäre Ammoniumverbindungen eingesetzt werden.

Es hat sich erfindungsgemäß als besonders bevorzugt erwiesen, wenn die erfindungsgemäßen Mittel frei sind von quartären Ammoniumverbindungen mit ein oder zwei (C₈ bis C₂₀)-Alkylresten.

Die erfindungsgemäßen Mittel liegen bevorzugt als Schaum vor. Hierzu werden die erfindungsgemäßen Mittel in einen Druckgasbehälter mit Abgabevorrichtung gefüllt ("Aerosolbehälter"). Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pump- oder Quetschsystem verteilt wird.

In der Regel werden alle Bestandteile des erfindungsgemäßen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

In der Ausführungsform als Aerosolschaum sind erfindungsgemäß geeignete Treibmittel beispielsweise ausgewählt aus N₂O, Dimethylether, CO₂, Luft, Alkanen mit 3 bis 5 Kohlenstoffatomen, wie Propan, n-Butan, iso-Butan, n-Pentan und iso-Pentan, und deren Mischungen. Gemäß der Ausführungsform eines Aerosolschaums werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Bevorzugt sind Dimethylether, Propan, n-Butan, iso-Butan und Mischungen daraus. Ganz besonders bevorzugt werden Mischungen von Propan und Butan als alleiniges Treibmittel verwendet im Gewichtsverhältnis Propan zu Butan von 70 zu 30 bis 15 zu 85. Diese Mischungen werden wiederum bevorzugt in den erfindungsgemäßen Mitteln in einer Menge von 2,0 bis 15,0 Gew.-% - bezogen auf das Gewicht des gesamten Mittels - eingesetzt. Unter Butan wird erfindungsgemäß n-Butan, iso-Butan und Gemische aus n-Butan und iso-Butan verstanden.

Bei Verwendung herkömmlicher Aerosolbehälter enthalten die erfindungsgemäßen Mittel das Treibmittel bevorzugt in Mengen von 1 bis 35 Gew.-%, bezogen auf das gesamte Mittel. Mengen von 2 bis 30 Gew.-%, insbesondere von 3 bis 15 Gew.-% sind besonders bevorzugt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus N₂O, Dimethylether, CO₂, Luft, Alkanen mit 3 oder 4 Kohlenstoffatomen (wie Propan, n-Butan, iso-Butan) sowie Mischungen daraus.

Ein zweiter Gegenstand der Erfindung ist die Verwendung eines kosmetischen Mittels des ersten Erfindungsgegenstandes zur temporären Umformung und/oder Formfixierung keratinischer Fasern, insbesondere menschlicher Haare.

Ein dritter Gegenstand der Erfindung ist ein Verfahren zur temporären Umformung keratinischer Fasern, insbesondere menschlicher Haare, dadurch gekennzeichnet, dass ein kosmetisches Mittel des ersten Erfindungsgegenstandes auf die keratinischen Fasern aufgebracht wird.

Es hat sich als bevorzugt herausgestellt, wenn die keratinischen Fasern nach der Einwirkung der kosmetischen Mittel des ersten Erfindungsgegenstandes nicht gespült und auf der Faser belassen werden.

Für den zweiten und dritten Erfindungsgegenstand gelten die bevorzugten Ausführungsformen des ersten Erfindungsgegenstandes *mutatis mutandis.*

Die nachfolgenden Beispiele sollen den Gegenstand der vorliegenden Erfindung erläutern ohne ihn in irgendeiner Weise zu beschränken.

### Beispiele:

Alle Mengenangaben sind, falls nicht anders gekennzeichnet, in Gewichtsprozent angegeben. Folgende erfindungsgemäßen Rezepturen wurden als Aerosolschaumprodukt hergestellt:

| Rohstoff | E1 | E2 | E3 | E4 | E5 | V1 | V2 |
|---|---|---|---|---|---|---|---|
| Luviskol® 60/40 W NP ¹ | 5,00 | 5,00 | 5,00 | 5,00 | 5,00 | 5,00 | 5,00 |
| Styleze® W 10 ² | 2,00 | 2,00 | 2,00 | 2,00 | 2,00 | 2,00 | 2,00 |
| Polyquaternium-16 | 1,00 | 1,00 | 1,00 | 1,00 | 1,00 | 1,00 | 1,00 |
| PEG-40 Hydrogenated Castor Oil | 0,20 | 0,20 | 0,20 | - | - | 0,40 | - |
| Eumulgin® B3 ³ | - | - | - | 0,20 | 0,20 | | |
| Eumulgin® B1 ⁴ | 0,20 | - | 0,10 | - | 0,10 | - | 0,10 |
| Eumulgin® O5 ⁵ | - | 0,20 | 0,10 | 0,20 | 0,10 | - | 0,10 |
| Wasser | <-----------------------------------ad 100---------------------------------> | | | | | | |

| | | | | | | | |
|---|---|---|---|---|---|---|---|
| ¹ Copolymer aus N-Vinylpyrrolidon und Vinylacetat ² Copolymer aus N-Vinylpyrrolidon, N,N-Dimethylaminopropylmethacrylamid und N,N-Dimethyl-N-dodecylammoniopropylmethacrylamid chlorid (10 Gew.-% Aktivsubstanz, INCI-Bezeichnung: Polyquaternium-55) (ISP) ³ Cetylstearylalkohol mit ca. 30 EO-Einheiten (INCI-Bezeichnung: Ceteareth-30) (Cognis) ⁴ Cetylstearylalkohol mit ca. 12-EO-Einheiten (INCI-Bezeichnung: Ceteareth-12) (Cognis) ⁵ Oleyl-Cetylalkohol mit ca. 5-EO-Einheiten auf pflanzlicher Basis (INCI-Bezeichnung: Oleth-5)(Cognis) | | | | | | | |

92 g der erfindungsgemäßen Rezepturen E1 bis E5 und der Vergleichsrezepturen V1 und V2 wurden in je einen Druckbehälter gefüllt, mit einem Ventil verschlossen und 8 g eines Treibmittels (Propan/Butan 15/85) eingefüllt. Die Mittel wurden jeweils als Schaum ausgebracht. Die erfindungsgemäßen Mittel zeigten einen Schaum, der sich schnell gebildet hat und auf der Handinnenfläche stabil war. Der Schaum des Mittels V1 bildete sich langsam aus. Das Mittel V2 lieferte einen Schaum, der auf der Handinnenfläche schnell brach und sich somit erschwert verwenden ließ.

Der Schaum wurde auf je eine angefeuchtete Haarsträhne zur Formfixierung aufgetragen und auf einen Spiralwickler gewickelt. Das mit den erfindungsgemäßen Mitteln behandelte Haar erhielt einen natürlichen Glanz, einen starken Halt und ein dauerhaftes Volumen. Haare, die mit dem Schaum des Mittels V1 behandelt wurden, wurden deutlich belastet und beschwert und hielten die Form schlechter.

## Patentansprüche

1. Kosmetisches Mittel zur kosmetischen Behandlung keratinischer Fasern, insbesondere menschlicher Haare, enthaltend in einem kosmetischen Träger
i) mindestens ein festigendes Polymer ausgewählt aus der Gruppe, die gebildet wird aus nichtionischen, festigenden Polymeren und kationischen, festigenden Polymeren, und
ii) 0,05 bis 0,4 Gew.-% - bezogen auf das Gesamtgewicht des Mittels - an Verbindungen der Formel (I), umfassend höchstens 20 Ethylenoxideinheiten
R¹-X¹-(O-CH₂CH₂)ₙ-OH (I)
worin
R¹ steht für einen linearen oder verzweigten, gesättigten oder ungesättigten Kohlenwasserstoffrest mit 8 bis 20 Kohlenstoffatomen,
X¹ steht für eine direkte Bindung oder eine Carbonylgruppe,
n steht für eine ganze Zahl von 1 bis 20,
und
iii) mindestens eine lineare oder verzweigte Verbindung der Formel (II) mit mindestens 30 Ethylenoxideinheiten 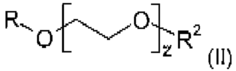 worin
R² für eine lineare oder verzweigte (C₇ bis C₂₁)-Alkylcarbonylgruppe, eine lineare oder verzweigte (C₇ bis C₂₁)-Alkenylcarbonylgruppe oder für einen linearen oder verzweigten, gesättigten oder ungesättigten (C₆ bis C₂₂)-Kohlenwasserstoffrest steht,
R steht für ein Wasserstoffatom oder eine Gruppe der Formel (III) 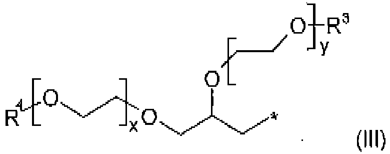
in der x und y unabhängig voneinander für eine ganze Zahl von 0 bis 80 stehen,
R³ und R⁴ unabhängig voneinander für ein Wasserstoffatom oder eine lineare oder verzweigte, gesättigte oder ungesättigte (C₇ bis C₂₁)-Alkylcarbonylgruppe stehen,
z steht für eine ganze Zahl von 0 bis 80
mit den Maßgaben, dass
- wenn R eine Gruppe der Formel (III) ist, mindestens eine Gruppe aus R², R³, R⁴ für eine lineare oder verzweigte (C₇ bis C₂₁)-Alkylcarbonylgruppe oder eine lineare oder verzweigte (C₇ bis C₂₁)-Alkenylcarbonylgruppe steht und die Summe x + y + z mindestens 30 beträgt,
- wenn R für ein Wasserstoffatom steht z eine ganze Zahl von 30 bis 80 bedeutet
und
iv) mindestens ein Treibmittel.

2. Kosmetisches Mittel nach einem der Ansprüche 1, **dadurch gekennzeichnet, dass** die festigenden Polymere in einer Menge von 0,1 Gew.-% bis 15,0 Gew.-%, insbesondere von 1,0 Gew.-% bis 12,0 Gew.-%, ganz besonders bevorzugt von 2,0 bis 10,0 Gew.-%, jeweils bezogen auf das Gewicht des erfindungsgemäßen kosmetisches Mittels, enthalten sind.

3. Kosmetisches Mittel nach einem der Ansprüche 1 oder 2, **dadurch gekennzeichnet, dass** gemäß Formel (I) X¹ für eine direkte Bindung steht.

4. Kosmetisches Mittel nach einem der Ansprüche 1 bis 3, **dadurch gekennzeichnet, dass** gemäß Formel (I) n für eine ganze Zahl von 1 bis 10 steht.

5. Kosmetisches Mittel nach einem der Ansprüche 1 bis 4, **dadurch gekennzeichnet, dass** die enthaltenen Verbindungen der Formel (I) ein Molekulargewicht von maximal 1200 g/mol, insbesondere maximal 750 g/mol, besitzen.

6. Kosmetisches Mittel nach einem der Ansprüche 1 bis 5, **dadurch gekennzeichnet, dass** als Verbindung der Formel (II) mindestens eine Verbindung der Formel (II-a) enthalten ist, 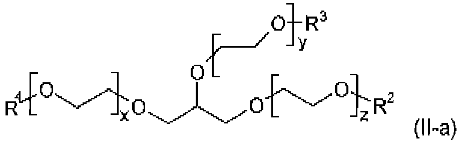 worin
R⁹, R³ und R⁴ stehen unabhängig voneinander für ein Wasserstoffatom, eine lineare oder verzweigte (C₇ bis C₂₁)-Alkylcarbonylgruppe, oder eine lineare oder verzweigte (C₇ bis C₂₁)-Alkenylcarbonylgruppe,
x, y und z stehen unabhängig voneinander für eine ganze Zahl von 0 bis 80
mit den Maßgaben, dass
- mindestens eine Gruppe aus R², R³, R⁴ von einem Wasserstoffatom verschieden ist und
- die Summe x + y + z mindestens 30 beträgt.

7. Kosmetisches Mittel nach Anspruch 6, **dadurch gekennzeichnet, dass** gemäß Formel (II-a) in der R², R³ und R⁴ stehen unabhängig voneinander für eine lineare oder verzweigte, gesättigte oder ungesättigte (C₇ bis C₂₁)-Alkylcarbonylgruppe.

8. Kosmetisches Mittel nach einem der Ansprüche 1 bis 7, **dadurch gekennzeichnet, dass** die Menge der Verbindungen der Formel (I) und der Verbindungen der Formel (II) in einem Gewichtsverhältnisbereich von 4 zu 1 bis 1 zu 4, insbesondere von 3 zu 1 bis 1 zu 3, liegen, liegen.

9. Kosmetisches Mittel nach einem der Ansprüche 1 bis 8, **dadurch gekennzeichnet, dass** der kosmetische Träger Wasser enthält.

10. Kosmetisches Mittel nach einem der Ansprüche 1 bis 9, **dadurch gekennzeichnet, dass** es als Schaum vorliegt.

## Claims

1. A cosmetic agent for cosmetically treating keratin fibers, in particular human hair, containing, in a cosmetic carrier,
i) at least one setting polymer selected from the group formed of nonionic setting polymers and cationic setting polymers, and
ii) from 0.05 to 0.4 wt.%, based on the total weight of the agent, of compounds of formula (I) comprising at most 20 ethylene oxide units
R¹-X¹-(O-CH₂CH₂)ₙ-OH (I)
in which
R¹ represents a linear or branched, saturated or unsaturated hydrocarbon functional group having 8 to 20 carbon atoms,
X¹ represents a direct bond or a carbonyl group,
n represents an integer from 1 to 20,
and
iii) at least one linear or branched compound of formula (II) having at least 30 ethylene oxide units 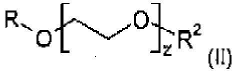 in which
R² represents a linear or branched (C₇ to C₂₁) alkylcarbonyl group, a linear or branched (C₇ to C₂₁) alkenylcarbonyl group, or a linear or branched, saturated or unsaturated (C₈ to C₂₂)-hydrocarbon functional group,
R represents a hydrogen atom or a group of formula (III) 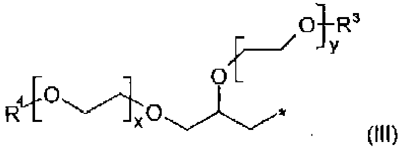
in which x and y represent, independently of one another, an integer from 0 to 80,
R³ and R⁴ represent, independently of one another, a hydrogen atom or a linear or branched, saturated or unsaturated (C₇ bis C₂₁) alkylcarbonyl group, z represents an integer from 0 to 80 with the provisos that,
- when R is a group of formula (III), at least one group of R², R⁵, R⁴ represents a linear or branched (C₇ to C₂₁) alkylcarbonyl group or a linear or branched (C₇ to C₂₁) alkenylcarbonyl group and the sum x + y + z is at least 30,
- when R represents a hydrogen atom, z represents an integer from 30 to 80
and
iv) at least one propellant.

2. The cosmetic agent according to claim 1, **characterized in that** the setting polymers are contained in an amount of from 0.1 wt.% to 15.0 wt.%, in particular from 1.0 wt.% to 12.0 wt.%, particularly preferably from 2.0 to 10.0 wt.%, in each case based on the weight of the cosmetic agent according to the invention.

3. The cosmetic agent according to one of claims 1 or 2, **characterized in that**, according to formula (I), X¹ represents a direct bond.

4. The cosmetic agent according to one of claims 1 to 3, **characterized in that**, according to formula (I), n represents an integer from 1 to 10.

5. The cosmetic agent according to one of claims 1 to 4, **characterized in that** the contained compounds of formula (I) have a molecular weight of at most 1200 g/mol, in particular at most 750 g/mol.

6. The cosmetic agent according to one of claims 1 to 5, **characterized in that** at least one compound of formula (II-a) is contained as the compound of formula (II), 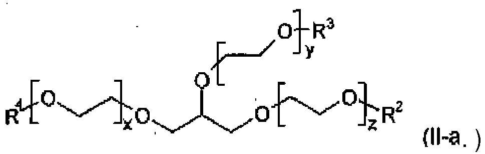 in which
R², R³ and R⁴ represent, independently of one another, a hydrogen atom, a linear or branched (C₇ to C₂₁) alkylcarbonyl group, or a linear or branched (C₇ to C₂₁) alkenylcarbonyl group,
x, y and z represent, independently of one another, an integer from 0 to 80 with the provisos that,
- at least one group of R², R³, R⁴ is different from a hydrogen atom and
- the sum x + y + z is at least 30.

7. The cosmetic agent according to claim 6, **characterized in that**, according to formula (II-a), in which R², R³ and R⁴ represent, independently of one another, a linear or branched, saturated or unsaturated (C₇ to C₂₁) alkylcarbonyl group.

8. The cosmetic agent according to one of claims 1 to 7, **characterized in that** the amount of the compounds of formula (I) and the compounds of formula (II) are in a weight ratio range of from 4:1 to 1:4, in particular from 3:1 to 1:3.

9. The cosmetic agent according to one of claims 1 to 8, **characterized in that** the cosmetic carrier contains water.

10. The cosmetic agent according to one of claims 1 to 9, **characterized in that** it is present as a foam.

## Revendications

1. Agent cosmétique destiné au traitement cosmétique des fibres kératiniques, en particulier des cheveux humains, contenant dans un support cosmétique
i) au moins un polymère stabilisant choisi dans le groupe constitué par les polymères fixants non ioniques et les polymères fixants cationiques, et
ii) 0,05 à 0,4 % en poids par rapport à la masse totale de l'agent, de composés de formule (I) comprenant au plus 20 unités d'oxyde d'éthylène
R¹-X¹-(O-CH₂CH₂)ₙ-OH (I)
dans lequel
R¹ représente un radical hydrocarboné linéaire ou ramifié, saturé ou insaturé, contenant 8 à 20 atomes de carbone,
X¹ représente une liaison directe ou un groupe carbonyle,
n est un nombre entier compris entre 1 et 20,
et
iii) au moins un composé linéaire ou ramifié de formule (II) contenant au moins 30 unités d'oxyde d'éthylène 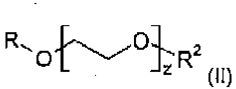 dans lequel
R² représente un groupe alkylcarbonyle linéaire ou ramifié (C₇ à C₂₁), un groupe alcénylcarbonyle linéaire ou ramifié (C₇ à ₂₁) ou un radical hydrocarboné linéaire ou ramifié, saturé ou insaturé (C₈ à C₂₂),
R représente un atome d'hydrogène ou un groupe de formule (III) 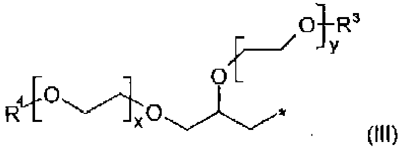
dans laquelle x et y représentent indépendamment l'un de l'autre un nombre entier de 0 à 80,
R³ et R⁴ représentent indépendamment l'un de l'autre un atome d'hydrogène ou un groupe alkylcarbonyle linéaire ou ramifié, saturé ou insaturé (C₁ à C₂₁), z représente un nombre entier de 0 à 80, à condition que
- si R est un groupe de formule (III), au moins un groupe comprenant R², R⁵ et R⁴ représente un groupe alkylcarbonyle (C₇ à C₂₁) linéaire ou ramifié ou un groupe alcénylcarbonyle (C₇ à G₂₁) linéaire ou ramifié, et la somme x + y + z est d'au moins 30,
- si R représente un atome d'hydrogène, z représente un nombre entier de 30 à 80
et
iv) au moins un agent propulseur.

2. Agent cosmétique selon l'une des revendications 1, **caractérisé en ce que** les polymères fixants sont présents dans une quantité allant de 0,1 % à 15,0 %, en particulier de 1,0 % à 12,0 %, de préférence de 2,0 % à 10,0 %, par rapport respectivement au poids de l'agent cosmétique selon l'invention.

3. Agent cosmétique selon l'une des revendications 1 ou 2, **caractérisé en ce que** selon la formule (I), X¹ représente une liaison directe

4. Agent cosmétique selon l'une des revendications 1 à 3, **caractérisé en ce que** selon la formule (I), n représente un entier de 1 à 10.

5. Agent cosmétique selon l'une des revendications 1 à 4, **caractérisé en ce que** les composés de formule (I) contenus ont une masse moléculaire de 1200 g/mol maximum, en particulier de 750 g/mol maximum.

6. Agent cosmétique selon l'une des revendications 1 à 5, **caractérisé en ce qu'**au moins un composé de formule (II-a) est présent comme composé de formule (II), 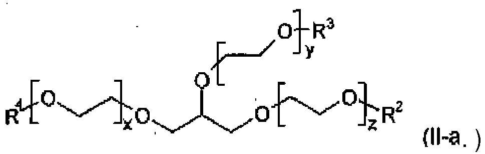 dans lequel
R², R³ et R⁴ représentent indépendamment les uns des autres un atome d'hydrogène, un groupe alkylcarbonyle linéaire ou ramifié (C₇ à C₂₁) ou un groupe alcénylcarbonyle linéaire ou ramifié (C₇ à C₂₁),
x, y et z représentent indépendamment les uns des autres un nombre entier de 0 à 80, à condition
- qu'au moins un groupe comprenant R², R³ et R⁴ soit différent d'un atome d'hydrogène, et
- que la somme x + y + z soit d'au moins 30.

7. Agent cosmétique selon la revendication 6, **caractérisé en ce que**, selon la formule (II-a) dans laquelle R², R³ et R⁴ représentent, indépendamment l'un de l'autre, un groupe alkylcarbonyle linéaire ou ramifié, saturé ou insaturé (C₇ à C₂₁).

8. Agent cosmétique selon l'une des revendications 1 à 7, **caractérisé en ce que** la quantité des composés de formule (I) et des composés de formule (II) se situe dans un intervalle de rapports pondéraux de 4 à 1 à 1 à 4, en particulier de 3 à 1 à 1 à 3.

9. Agent cosmétique selon l'une des revendications 1 à 8, **caractérisé en ce que** le support cosmétique contient de l'eau.

10. Agent cosmétique selon l'une des revendications 1 à 9, **caractérisé en ce qu'**il se présente sous forme de mousse.
